(19) Europäisches Patentamt
European Patent Office
Office européen des brevets

(11) Veröffentlichungsnummer: **0 207 416**
**B1**

(12) # EUROPÄISCHE PATENTSCHRIFT

(45) Veröffentlichungstag der Patentschrift: **16.08.89**

(51) Int. Cl.⁴: **C 07 D 277/82**

(21) Anmeldenummer: **86108518.1**

(22) Anmeldetag: **23.06.86**

(54) **Verfahren zur Herstellung von 2-Aminobenzthiazolen.**

(30) Priorität: **27.06.85 DE 3522941**

(43) Veröffentlichungstag der Anmeldung: **07.01.87 Patentblatt 87/2**

(45) Bekanntmachung des Hinweises auf die Patenterteilung: **16.08.89 Patentblatt 89/33**

(84) Benannte Vertragsstaaten: **BE CH DE FR GB IT LI**

(56) Entgegenhaltungen:
**US-A- 4 363 913**

**CHEMICAL ABSTRACTS, Band 96, Nr. 25, 21. Juni 1982, Seite 744, Zusammenfassung Nr. 217832q, Columbus, Ohio, US; & JP-A-82 09 774**

**Die Akte enthält technische Angaben, die nach dem Eingang der Anmeldung eingereicht wurden und die nicht in dieser Patentschrift enthalten sind.**

(73) Patentinhaber: **HOECHST AKTIENGESELLSCHAFT, Postfach 80 03 20, D-6230 Frankfurt am Main 80 (DE)**

(72) Erfinder: **Rentél, Heinz, Dr., Höhenstrasse 45, D-6242 Kronberg im Taunus (DE)**
Erfinder: **Papenfuhs, Theodor, Dr., Heinrich-Bleicher-Strasse 40, D-6000 Frankfurt am Main 50 (DE)**

EP 0 207 416 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Es ist bekannt, 2-Aminobenzthiazole durch Cyclisierung entsprechender Phenylthioharnstoffe in 85- bis 100%iger Schwefelsäure in Gegenwart katalytischer Mengen Brom, Bromwasserstoff, Natrium-, Kalium- oder Ammoniumbromid bei Temperaturen von 20 - 130°C herzustellen (Japanische Patentanmeldung Sho 57-9774; US-PS 4 363 913). Die Reaktion verläuft jedoch nicht einheitlich, so daß durch Nebenreaktionen (Sulfierung, Bromierung, Hydrolyse des Phenylthioharnstoffs mit zum Teil nachfolgenden Substitutionen der erstgenannten Art) qualitativ nicht befriedigende Produktgemische entstehen, die eine aufwendige Reinigung erfordern und (bei geringen Löslichkeitsunterschieden der Ziel- und Nebenprodukte) schlechte Ausbeuten ergeben.

Es wurde nun überraschenderweise gefunden, daß man 2-Aminobenzthiazole der allgemeinen Formel (1)

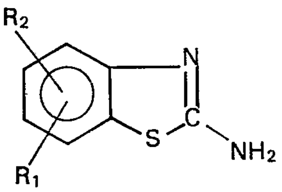

(1)

in welcher $R_1$ und $R_2$ Wasserstoff- oder Halogenatome, wie Fluor-, Chlor-, Brom- oder Jodatome, oder $Alkyl_{C_1-C_4}$, $Alkoxy_{C_1-C_4}$ oder Nitrogruppen bedeuten, unter Vermeidung von Nebenreaktionen in hohen Ausbeuten und in hoher Reinheit durch Umsetzung von Phenylthioharnstoffen der allgemeinen Formel (2)

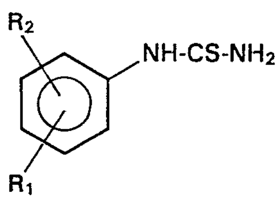

(2)

in welcher $R_1$ und $R_2$ die vorstehend genannten Bedeutungen haben, in der an sich bekannten Weise (siehe weiter oben) erhalten kann, wenn zusätzlich fein verteilte, unter den Reaktionsbedingungen inerte Feststoffe großer spezifischer Oberfläche zugegen sind.

Zu geeigneten Feststoffen der genannten Art zählen beispielsweise amorphe Kieselsäuren (Kieselgur), die z.B. unter den Namen ®Celite, ®Dicalite, ®Clarcel, ®VDK-Füllstoffgur, ®Fina/Optima-Filterhilfsmittel im Handel sind, hochaktive Bleicherden, beispielsweise hergestellt aus dem Tonmineral Montmorrillonit (im Handel unter den Namen ®Terrana, ®Tonsil oder ®Clarit), vulkanische Liparit- oder Quarzporphyrgläser («Perlite»), Fällungskieselsäuren (im Handel z.B. unter den Namen ®Ultrasil, ®Silcasil, ®Vulcasil, ®Silcatron oder ®Sipernat), Kieselgele (z.B. als Handelsmarken ®Syloid, ®Gasil, ®Lucilite, ®Silicon, ®Diamantgel) oder pyrogene Kieselsäuren (im Handel z.B. unter den Namen ®Aerosil, ®Cab-O-Sil, ®HDK) sowie Aktivkohlen (z.B. als Handelsmarken ®Actibon, ®Carboraffin, ®Norit, ®Acticarbone, ®Alcarbon, ®Brilonit, ®Darco, ®Eponit).

Das erfindungsgemäße Verfahren wird im einzelnen in der Weise durchgeführt, daß man den Phenylthioharnstoff der genannten Formel (2) in der 1- bis 10fachen Gewichtsmenge Schwefelsäure einer Konzentration von 70 bis 100% unter Rühren löst, anschließend den erfindungsgemäßen feinverteilten Feststoff in einer Menge von 0,1 bis 0,01 Gewichtsprozent, bezogen auf den eingesetzten Phenylthioharnstoff der Formel (2), zufügt und nach Zugabe des Katalysators in Form von Brom, Bromwasserstoff, Natrium-, Kalium- oder Ammoniumbromid, die Cyclisierung bei Temperaturen von 20°C bis 130°C, vorzugsweise bei 40°C bis 90°C durchführt.

Das entstandene Abgas ($SO_2$) wird in einer mit Alkalilauge betriebenen Gaswäsche zu wiederverwendbarer Bisulfitlösung umgesetzt.

Der Fortgang der Cyclisierungsreaktion ist durch analytische Methoden, beispielsweise dünnschicht-, gas- oder flüssigchromatografische Verfahren, leicht zu verfolgen.

Nachdem alles Ausgangsmaterial umgesetzt ist, wird das Cyclisierungsgemisch mit Wasser verdünnt, von ungelösten Anteilen durch Klärfiltration befreit und das gebildete 2-Aminobenzthiazol der genannten Formel (1) in Form seines Sulfats durch Abkühlen ausgeschieden oder als freie Base durch Zusatz ausreichender Mengen eines Alkalimetallhydroxids oder mit wäßriger Ammoniaklösung ausgefällt und durch Filtration isoliert.

Die nachstehenden Ausführungsbeispiele sollen das erfindungsgemäße Verfahren näher erläutern, ohne es darauf zu beschränken.

*Beispiel 1*

In einem emaillierten Gefäß werden 150 kg konz. Schwefelsäure vorgelegt. Unter Rühren trägt man 49,3 kg 4-Nitrophenylthioharnstoff bei Raumtemperatur ein, verrührt und fügt dann 2 kg Perlite zu. Anschließend werden 2 kg Ammoniumbromid in Anteilen zugegeben und die Reaktion bei 100°C - 105°C in 2,5 - 3 Stunden zu Ende geführt. Ist kein 4-Nitrophenylthioharnstoff mehr nachweisbar, wird die Reaktionsmischung auf 750 kg Wasser gedrückt und bei etwa 95°C geklärt. In das geklärte Filtrat läßt man unter Kühlung 160 kg 25%ige Ammoniaklösung einlaufen bis ein pH-Wert von 3,5 erreicht ist, filtriert bei etwa 70°C das ausgefallene 2-Amino-6-nitrobenzthiazol ab und wäscht mit heißem Wasser neutral.

Nach dem Trocknen erhält man 44,9 kg 2-Amino-6-nitrobenzthiazol (Schmelzpunkt 247°C), was einer Ausbeute von 91,0% der Theorie entspricht. Das Produkt ist in 80%iger Phosphorsäure klar löslich. Die HPLC-Analyse zeigt keine Spur einer Verunreinigung. («HPLC» = high performance liquid chromatography.)

*Beispiel 2*

In 150 kg konzentrierter Schwefelsäure werden 49,3 kg 4-Nitrophenylthioharnstoff unter Rühren gelöst und 2,5 kg feingemahlene Kieselgur zugesetzt. Anschließend fügt man 2,5 kg Brom innerhalb einer

Stunde zu, wobei die Temperatur 45°C - 50°C erreicht. Anschließend wird auf 95°C - 100°C hochgeheizt und etwa 2 - 3 Stunden bei dieser Temperatur gehalten, bis kein 4-Nitrophenylthioharnstoff mehr nachweisbar ist. Dann wird das Reaktionsgemisch auf 800 kg Wasser gegeben, bei etwa 95°C geklärt und danach mit wäßriger Natriumhydroxidlösung auf einen pH-Wert von 3,5 eingestellt. Das ausgefallene 2-Amino-6-nitrobenzthiazol wird heiß abfiltriert und mit heißem Wasser neutral gewaschen.

Nach dem Trocknen erhält man ein 2-Amino-6-nitrobenzthiazol vom Schmelzpunkt 247°C, das in 80%iger Phosphorsäure bei 50°C klar löslich ist. Die HPLC-Analyse zeigt keine Spur einer Verunreinigung. Gewonnen werden 44,6 kg, was einer Ausbeute von 90,4% der Theorie entspricht.

*Beispiele 3 - 10*

Man verfährt, wie in Beispiel 1 beschrieben, ersetzt jedoch den 4-Nitrophenylthioharnstoff bzw. den erfindungsgemäßen Feststoff durch die in der nachfolgenden Tabelle genannten Produkte:

| Beispiel Nr. | Thioharnstoff [Verbindung der Formel (2)] | Erfindungsgemäßer Feststoff | Endprodukt | Ausbeute/Reinheit (HPLC) |
|---|---|---|---|---|
| 3 | 46,6 kg 2-Chlorphenylthioharnstoff | 2,5 kg Celite | 2-Amino-4-chlorbenzthiazol | 92% d.Th./ 99%ig |
| 4 | 50 kg 2-Methyl-4-chlorphenylthioharnstoff | 3 kg Tonsil | 2-Amino-4-methyl-6-chlorbenzthiazol | 91,5% d.Th./ 99,1%ig |
| 5 | 45,5 kg 4-Methoxyphenylthioharnstoff | 4,5 kg Perlite | 2-Amino-6-methoxybenzthiazol | 90,5% d.Th./ 98,6%ig |
| 6 | 55,3 kg 3,4-Dichlorphenylthioharnstoff | 3,5 kg Celite | 2-Amino-5(7),6-dichlorbenzthiazol (Isomerengemisch) | 96,1% d.Th./ 99,4%ig |
| 7 | 55,3 kg 3,4-Dichlorphenylthioharnstoff | 2,0 kg A-Kohle | 2-Amino-5(7),6-dichlorbenzthiazol (Isomerengemisch) | 95,8% d.Th./ 98,9%ig |
| 8 | 46,6 kg 4-Chlorphenylthioharnstoff | 2,0 kg Kieselgur | 2-Amino-6-chlorbenzthiazol | 94,8% d.Th./ 99,2%ig |
| 9 | 55,3 kg 2,4-Dichlorphenylthioharnstoff | 3,0 kg Carcel | 2-Amino-4,6-dichlorbenzthiazol | 95,0% d.Th./ 99,4%ig |
| 10 | 55,3 kg 2,4-Dichlorphenylthioharnstoff | 3,0 kg Perlite | 2-Amino-4,6-dichlorbenzthiazol | 96,1% d.Th./ 99,2%ig |

*Vergleichsbeispiel*

Verfährt man, wie in Beispiel 1 beschrieben, jedoch ohne Zusatz von erfindungsgemäßem Feststoff (Perlite), so erhält man ein 6-Nitro-2-aminobenzthiazol vom Schmelzpunkt 238°C - 240°C, welches in 80%iger Phosphorsäure bei 60°C nicht klar löslich ist und folgende Verunreinigungen enthält (HPLC, gemessen gegen authentische Vergleichssubstanzen):

1,6% 4-Nitranilin
1,8% 2-Brom-4-nitranilin, 2,6-Dibrom-4-nitranilin
0,6% 4-Nitrophenylthioharnstoff.

**Patentansprüche**

1. Verfahren zur Herstellung von 2-Aminobenzthiazolen der allgemeinen Formel (1)

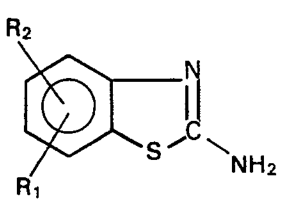

(1)

in welcher $R_1$ und $R_2$ Wasserstoff- oder Halogenatome, oder Alkyl$_{C_1-C_4}$, Alkoxy$_{C_1-C_4}$ oder Nitrogruppen bedeuten, durch Umsetzung von Phenylthioharnstoffen der allgemeinen Formel (2)

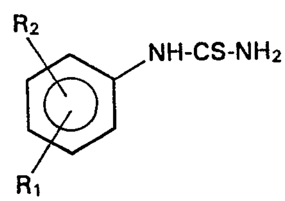

(2)

in welcher $R_1$ und $R_2$ die vorstehend genannten Bedeutungen haben, in 70- bis 100%iger Schwefelsäure in Gegenwart katalytischer Mengen von Brom, Bromwasserstoff, Natrium-, Kalium- oder Ammoniumbromid bei Temperaturen von 20°C bis 130°C, dadurch gekennzeichnet, daß man die Umsetzung in Gegenwart eines feinverteilten, unter den Reaktionsbedingungen inerten Feststoffes großer spezifischer Oberfläche durchführt.

2. Verfahren nach Anspruch 1, dadurch gekennzeichnet, daß man die Umsetzung in Gegenwart von amorpher, pyrogener oder Fällungs-Kieselsäure,

Kieselgel, Kieselgur, Bleicherde, vulkanischen Liparit- oder Quarzporphyrgläsern oder Aktivkohle als Feststoff durchführt.

3. Verfahren nach Anspruch 1, dadurch gekennzeichnet, daß man die Umsetzung bei 40°C bis 90°C durchführt.

4. Verfahren nach Anspruch 1, dadurch gekennzeichnet, daß man die Umsetzung in Gegenwart von 0,1 bis 0,01 Gewichtsprozent Feststoff, bezogen auf eingesetzten Phenylthioharnstoff der Formel (2), durchführt.

**Claims**

1. A process for preparing 2-aminobenzthiazoles of the formula (1)

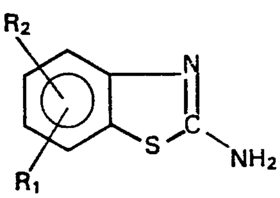

(1)

in which $R_1$ and $R_2$ denote hydrogen or halogen atoms or alkyl$_{C_1\text{-}C_4}$, alkoxy$_{C_1\text{-}C_4}$ or nitro groups, by reacting phenylthioureas of the formula (2)

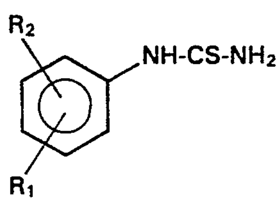

(2)

in which $R_1$ and $R_2$ have the abovementioned meanings, at temperaturs of 20°C to 130°C in 70 - 100% strength sulfuric acid in the presence of catalyst amounts of bromine, hydrogen bromide, sodium bromide, potassium bromide or ammonium bromide, which comprises carrying out the reaction in the presence of a finely divided solid which has a large specific surface area and is inert under the reaction conditions.

2. The process as claimed in claim 1, wherein the reaction is carried out in the presence of amorphous, pyrogenic or precipitated silica, silica gel, kieselguhr, bleaching earth, vulcanic liparite or quartz porphyric glasses or activated carbon as the solid.

3. The process as claimed in claim 1, wherein the reaction is carried out at 40°C to 90°C.

4. The process as claimed in claim 1, wherein the reaction is carried out in the presence of 0.1 to 0.01 percent by weight of solid, based on starting phenylthiourea of the formula (2).

**Revendications**

1. Procédé de préparation d'amino-2 benzthiazoles répondant à la formule (1):

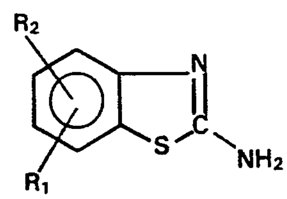

(1)

dans laquelle $R_1$ et $R_2$ représentent chacun un atome d'hydrogène ou d'halogène, un alkyle en $C_1$-$C_4$, un alcoxy en $C_1$-$C_4$ ou un radical nitro, par réaction de phényl-thio-urées répondant à la formule générale (2):

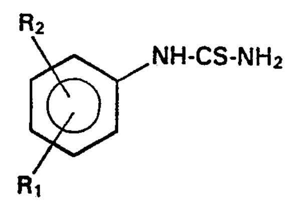

(2)

dans laquelle $R_1$ et $R_2$ ont les significations qui leur ont été données ci-dessus, dans un acide sulfurique d'une concentration comprise entre 70 et 100%, en présence de quantités catalytiques de brome, de bromure d'hydrogène ou de bromure de sodium, de potassium ou d'ammonium, à des températures comprises entre 20 à 130°C, procédé caractérisé en ce qu'on effectue la réaction en présence d'une matière solide finement divisée, à grande surface spécifique, qui est inerte dans les conditions de la réaction.

2. Procédé selon la revendication 1, caractérisé en ce que'on effectue la réaction en présence d'une silice amorphe, d'une silice pyrogène, d'une silice obtenue par précipitation, d'un gel de silice, de kieselguhr, d'une terre décolorante, de verres volcaniques du type de la liparite ou du porphyre quartzifère, ou d'un charbon actif, comme matière solide.

3. Procédé selon la revendication 1, caractérisé en ce que'on effectue la réaction à une température de 40 à 90°C.

4. Procédé selon la revendication 1, caractérisé en ce que'on effectue la réaction en présence de 0,1 à 0,01% en poids de la matière solide, par rapport à la phényl-thio-urée de formule (2) mise en jeu.